# EUROPEAN PATENT SPECIFICATION

(11) **EP 1 349 517 B1**
(45) Date of publication and mention of the grant of the patent: **17.01.2007**
(21) Application number: 01989976.4
(22) Date of filing: 02.11.2001
(51) Int. Cl.: A61F 2/90

(54) **STENT FOR TREATING IN-STENT RESTENOSIS**
STENT ZUR BEHANDLUNG VON RESTENOSIS IN EINEM STENT
PROTHESE ENDOVASCULAIRE POUR RESTENOSE EN PROTHESE

(30) Priority: 12.01.2001 US 681118
(43) Date of publication of application: 08.10.2003
(73) Proprietor: Boston Scientific Limited, St. Michael, Barbados, West Indies (BB)
(72) Inventor: SIMSO, Eric, J., Excelsior, MN 55331 (US); MILLER, Matthew, J., White Bear Lake, MN 55110 (US)
(74) Representative: Graalfs, Edo
(86) International application number: PCT/US2001/046925
(87) International publication number: WO 2002/054985

(56) References cited:
- EP-A- 0 876 806
- US-A- 5 853 419

## Description

### Background of the Invention

A stenosis of an artery is a constriction or narrowing of the artery. The stenosis may be as a result of the buildup up of cholesterol, fat or other substances. Regardless of the cause of the stenosis, a stenosis can be life threatening. Stenoses of coronary arteries, for example, can diminish the flow of blood to the heart leading to heart damage and, possibly, death.

A number of methods have been developed for widening arteries which have become stenosed. These methods include stenting, percutaneous transluminal coronary angioplasty (PTCA), coronary artery bypass graft (CABG) procedures and the use of drugs and/or radiation.

Of the various methods of widening arteries which have become stenosed, stenting has proven to be of particular value in lowering the rate of restenosis or formation of new stenoses following the procedure. Even stented regions of a vessel, however, can restenose.

Restenosis only occurs in a fraction of patients. It can, however, be difficult to treat in patients in whom it occurs. Restenossis typically occurs within six months to one year of initial treatment of the vessel.

One inch should be replaced with 2.5 cm.

A number of different techniques have been devised to reduce the likelihood of restenosis in stented regions of a vessel. These techniques include treating the patient with anticoagulant and antiplatelet drugs and smooth muscle cell inhibitors. Direct delivery of drugs to the affected vessel is addressed in a plethora of patents including US 5,861,168 which discloses the use of a stent bearing a nitric oxide precursor agent and US 5,800,507 which discloses the use of a stent bearing fibrin. Other methods of treatment include delivery of radioactive substances to the affected region of the body as is disclosed in US 5,871,437, US 5,059,166 and US 6,099,455. The use of ultrasonic energy in reducing the likelihood of restenosis is disclosed US 5,836,896. US 5 853 419 suggests stents with a wall- thickness of 0.001 inches or higher.

There remains a need for providing novel methods of dealing with restenosis in stented regions of a vessel as well as novel apparatuses for accomplishing the same.

The technical problem of the invention is to provide a stent to reduce the likelihood of restenosis in stented regions of a vessel.

The problem is solved by a second stent according to claim 1.

### Summary of the Claimed Embodiments of the Invention

At least a portion of the second stent has a wall thickness of less than 0.001 inch.

The first stent may be any suitable stent known in the art.

The second stent may be any suitable design known in the art. Examples of stent designs include stents having one or more segments of interconnected struts and stents having one or more segments with cells and openings therethrough. Desirably, where the second stent has a tubular surface with openings, the second stent when expanded has a ratio of the area of the surface to the area of the openings of at least 3: 7, desirably at least 1:1 and more desirably, at least 3: 2. The second stent may be helical or non-helical.

The second stent may be made of any suitable material whether polymeric or metal or a combination of the two or otherwise. Where the second stent is made of foil, desirably the second stent is only one layer of foil thick. The second stent may be mechanically expandable or self-expanding. In the latter case, the second stent is desirably made of a shape memory material.

Optionally, the second stent may comprise a liner or a bioabsorbable membrane. Where a liner is provided, the liner may be made of any suitable graft material whether bioabsorbable or not. An example of a suitable non-bioabsorbable material is expanded PTFE (ePTFE). The bioabsorbable membrane or liner may further comprise a treatment agent.

In one embodiment, the second stent comprises at least a first support and a second support, and a liner extending between the first support and the second support. Optionally, the first and second supports may be hoops.

The second stent may be used in a manner to frictionally engage the first stent. Optionally, the second stent may be attached to the first stent, for example, via one or more hooks. The second stent may also be attached to the first stent by being bonded thereto. Where the second stent is self-expanding, it may frictionally engage the first stent by self-expanding against the first stent and applying an outward force against the first stent.

The invention may be used in a method of stenting a previously stented region of a bodily vessel where the stented region of the bodily vessel has a first stent therein with a lumen therethrough. The method comprises the steps of disposing a second stent in the lumen of the first stent and implanting the second stent in the lumen of the first stent. At least a portion of the second stent has a wall thickness of 0.002 inches or less, more desirably, 0.001 inches or less, and most desirably, 0.0005 inches or less. The second stent may be of any suitable design, as discussed above and may be mechanically expandable or self-expanding. The method may be used to treat a restenosed, previously stented area of a vessel.

In yet another embodiment, the invention may be used in a method of stenting a previously stented region of a bodily vessel. In accordance with the method, a second stent is disposed in the lumen of an already implanted first stent and implanted therein. The second stent has a tubular surface with openings therethrough. When the second stent is expanded, the ratio of the area of the surface to the area of the openings is at least 3:7, more desirably 1:1 and most desirably, 3:2. The method may be used to treat a restenosed, previously stented area of a vessel.

Such methods may be carried out at any point subsequent to implantation of the first stent using any suitable first stent and second stent as discussed above. Desirably, the first stent will have been implanted in the bodily vessel for a period of time in excess of one month and more desirably, for a period of time in excess of one half year prior to insertion of the second stent. Most desirably, the first stent will have been implanted in the bodily vessel for a period of time in excess of one year.

Such methods may be preceded and/or followed by one or more steps in which the stented region of the vessel is treated to reduce restenosis.

A detailed description of the invention in its various embodiments is provided below.

### Brief Description of the Figures

Fig. 1 shows a perspective view of the inventive stent combination in a vessel with parts cut away.
Fig. 2 shows a cross-section of the inventive combination of Fig. 1 taken along line 2-2.
Fig. 3 shows a perspective view of a stent.
Fig. 4 shows a perspective view of a stent.
Fig. 5 shows a stent with a membrane.
Fig. 6 shows a stent having a plurality of supports and a liner.
Fig. 7 shows a first and second stent with hook

### Detailed Description of the Invention

While this invention may be embodied in many different forms, there are described in detail herein specific preferred embodiments of the invention. This description is an exemplification of the principles of the invention and is not intended to limit the invention to the particular embodiments illustrated.

For the purposes of this disclosure, unless otherwise indicated, like reference numerals in the figures shall refer to like structures.

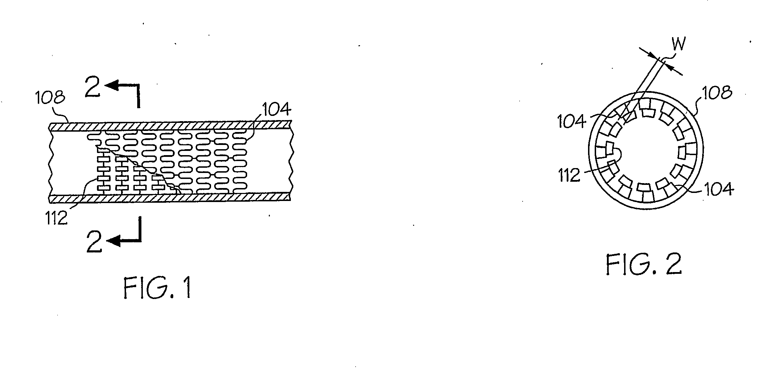

Turning to Figs. 1 and 2, the invention may be used in a combination of a first expanded stent 104 and a second expanded stent 112 disposed within first stent 104 where at least a portion of second stent 112 has a wall thickness 116 less than, 0.001 inches. Even more desirably, the wall thickness is 0.0005 inches or less and most desirably, the wall thickness is 0.00025 inches or less. The first expanded stent and second expanded stent may optionally be implanted in a bodily vessel 108.

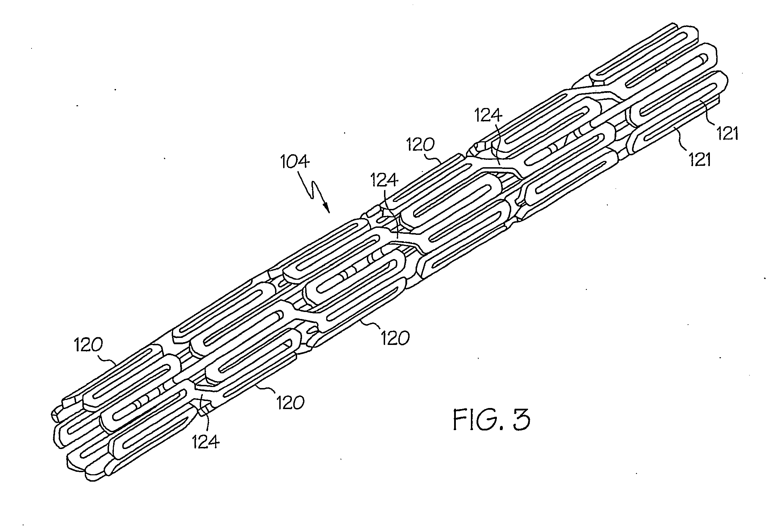

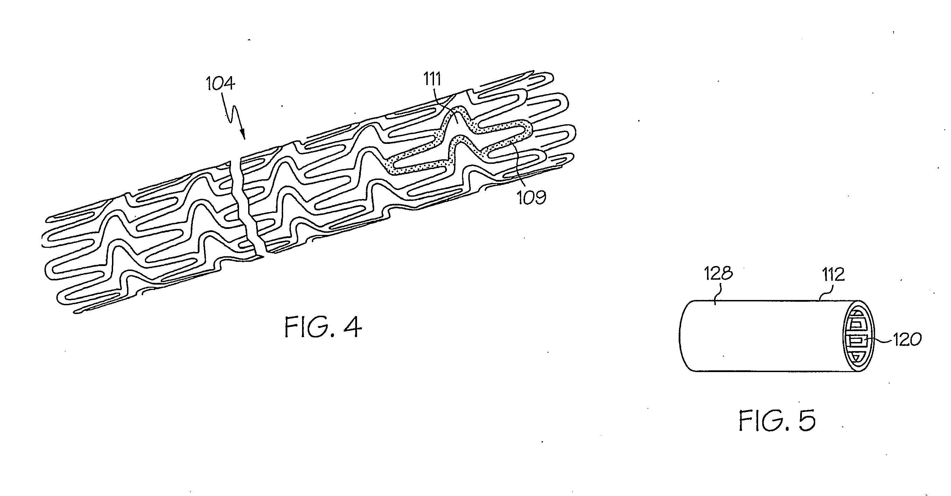

First stent 104 may be any suitable stent for implantation in a bodily vessel as known in the art. Desirably, the stent comprises one or more serpentine segments 120 as shown in Fig. 3. The stent of Fig. 3 comprises a plurality of interconnected segments joined by angled connectors 124. The stent may also be formed of a plurality of serpentine segments 120. Each serpentine segment comprises a plurality of connected struts 121. Adjacent serpentine segments 120 are joined by connectors 124 having one or more bends therein. The ends of the connectors may be circumferentially displaced as shown in Fig. 3 or circumferentially aligned with one another. Another example of a stent is shown in Fig. 4. Stent 104, as shown in Fig. 4, includes a plurality of interconnected closed cells 109 (one of which is shown shaded). Each closed cell 109 includes an opening 111 therethrough. Stents having at least one segment having closed cells therein, are also disclosed in US 5,733,303, US 6,059,810 and US 6,033,433. Other examples of first stents include spiral or helical stents, stents having a constant diameter when expanded, stents at least a portion of which taper whether an end portion, a middle portion or any other portion and conical stents. The first stent may include one or more radiopaque portions.

First stent 104 may be mechanically expandable, for example balloon expandable or may be self-expandable.

The first stent may be made of any suitable metallic material or combination of metallic materials including tungsten, aluminum, metal alloys, stainless steel, tantalum, rhenium, titanium, or other types of metallic materials. Alternatively, non-metallic materials, composites of metallic and non-metallic materials or other composites may be used. Metal-plastic, metal-ceramic composites may be used, and non-metals such as rubbers, ceramics, plastics or other polymers. The stent may be optionally made of shape memory materials, whether metal, polymeric or otherwise.

Second stent 112 desirably is self-expandable and may be made of any suitable material including those specifically disclosed above. Desirable shape memory materials include Nitinol as disclosed in WO 96/26689 and US 6,059,810. The invention also contemplates second stents which mechanically expandable, for example, by balloon. An example of such a stent is disclosed in US 5,733,303. Second stent 112 may also be a foil stent. Where a foil stent is used, the foil stent is desirably only one layer of foil thick. More information about foil stents may be found in US 6,120,535.

Second stent 112 may be of any suitable design including the designs discussed above for the first stent. Desirably, the stent comprises one or more serpentine segments such as those shown at 120 in Fig. 3. Adjacent segments may be connected via angled connectors as shown at 124 in Fig. 3. As with the first stent, the second stent may also be formed of a plurality of serpentine segments joined by connectors having one or more bends therein. The ends of the connectors may be circumferentially aligned with one another or circumferentially displaced from one another. The second stent may include a plurality of interconnected closed cells such as those shown at 109 in Fig. 4. Stents having at least one segment having closed cells therein, are also disclosed in US 5,733,303, US 6,059,810 and US 6,033,433.

The term "maximum PIN opening" is used herein to describe micro openings in which the dimensions specify the largest which can be passed through the cell opening. This applies as noted above to the expanded stent configuration. Typically, as a stent is expanded to larger diameters, the opening becomes larger. It is believed that using a maximum PIN opening specification that the concept of the present invention may be more readily applicable to stents of either open or closed cell geometries.

Yet other examples of a second stent include spiral or helical stents, stents having a constant diameter when expanded, stents at least a portion of which taper whether an end portion, a middle portion or any other portion and conical stents.

The radiopaque portions of the second stent when expanded may line up with one or more radiopaque portions of the first stent or may displaced from any radiopaque portions of the first stent.

As shown in Fig. 5, second stent 112 may comprise an optional liner 128 disposed on the outer surface of the stent or stent segments 120. The invention also contemplates the presence of an optional liner in the interior of the stent. Liner 128 may be any suitable graft material including expanded PTFE (ePTFE). Liner 128 may also be a bioabsorbable membrane.

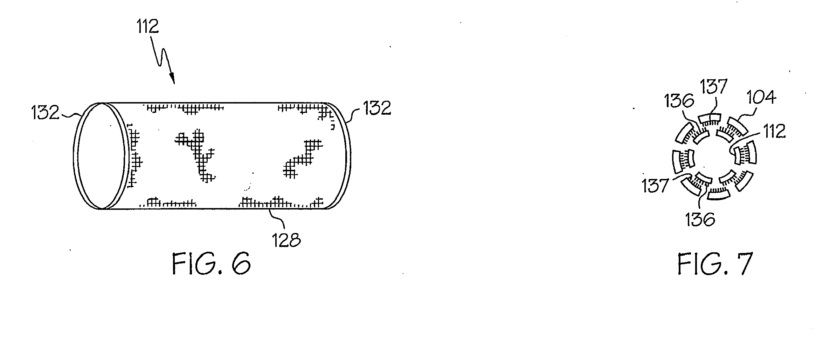

Second stent 112 may also comprise a plurality of supports 132 with a liner 128 extending therebetween, as shown in Fig. 6. Desirably, supports 132 are in the form of hoops. Liner 128 has a thickness of less than 0.002 inches and more desirably, less than 0.001 inches. Even more desirably, the thickness is 0.0005 inches or less and most desirably, the thickness is 0.00025 inches or less. Liner 128 may be made of foil or any suitable graft material including bioabsorbable materials and ePTFE.

The second stent may optionally be attached to the first stent. The attachment may be through one or more hooks which may be secured to the first stent. Optionally, the first stent may comprise a plurality of loops for receiving the hooks. Desirably, the hooks and loops operate similar to VELCRO^{™}. As shown in Fig. 7, hooks 136 extending from second stent 112 engage loops 137 extending from first stent 104. The hooks may extend from one of the first or the second stent with the loops extending from the other stent. The second stent may also be bonded to the first stent via the use of adhesives.

The second stent may apply an outward force against the first stent and form a friction fit with the first stent such as is the case with a self-expanding stent

Where second stent 112 has a tubular surface with openings 111 therethrough, as shown for example in Fig. 4, desirably, the ratio of the area of the surface to the area of the openings at least 3:7 when the second stent is expanded. More desirably, the ratio of the area of the surface to the area of the openings when the second stent is expanded is at least 1:1. Even more desirably, the ratio of the area of the surface to the area of the openings is at least 3:2. Providing a high ratio of surface area to the area of the openings is desirable in preventing additional restenosis.

The invention may be used in a combination of a first stent implanted in a bodily vessel and a second stent disposed within the first stent, as shown, for example, in Fig. 1, where the second stent has a tubular surface with openings therethrough. When the second stent is expanded, the ratio of the area of the surface to the area of the openings at least 3:7. More desirably, the ratio of the area of the surface to the area of the openings is at least 1:1. Even more desirably, the ratio of the area of the surface to the area of the openings is at least 3 :2.

Desirably, in any of the above embodiments, second stent 112 comprises a treatment agent. The treatment agent may be applied to any portion or the entirety of the stent. In one embodiment, where the second stent comprises a liner, the treatment agent may be applied to the liner. In another embodiment, where the second stent comprises a plurality of struts and/or segments, the treatment agent may be applied to at least a portion of the struts and/or segments.

Suitable treatment agents include hydrophilic drugs such as heparin or hirudin to protect against coagulation. Hydrophobic drugs such as prostaglandins or aspirin and vitamin E may be used to protect against platelet activation. Vitamin E and other anti oxidants such as sodium ascorbate, phendies, carbazoles, and tocotrienols may be used to protect against oxidation. The treatment agent may also include prostaglandins PGI2 or PGE2, RGD peptide, thrombomodulin, TPA (Tissue Plasminogen Activator) and Urokinase as well as other bioactive proteins. Gene therapy agents such as antiplatelet and antibody fragments, for example GB2B3A may also be provided. Other suitable agents include nitric oxide precursor agents, fibrin, Taxol and ticlopidine. Any of the treatment agents disclosed in US 6,074,659 US 6,120,847, US 5,861,168, US 5,800,507 and US 5,693,085 and any of the methodologies disclosed therein for delivering treatment agents may be used. More generally, the treatment materials may include the known antithrombic agents, anti-angiogenesis agents, antibacterial and/or antimicrobial agents and antifungal agents.

The treatment agent may also be radioactive. The radioisotope used in the treatment agent may be an alpha, beta or gamma emitter. Desirably, the half-life of the radioisotope is between 10 hours and 100 days. More desirably, the radioisotope will be a beta emitting isotope such as phosphorous 32, with a 14.3 day half-life and no gamma rays. Additional details concerning such a treatment agent may be found in US 5,722,984 and US 5,871,437. Other suitable radioisotopes for use as treatment agents include the beta emitting isotope gold 198 (half-life 2.7 days) as disclosed in US 5,059,166, beta emitting strontium-89 with a half-life of approximately 50.5 days as disclosed in US 6,129,658, sulfur-35 as disclosed in US 5,919,126 and beta particle-emitting radioactive metals selected from the group consisting of rhenium, copper, dysprosium, yttrium, holmium, praseodymium, lanthanum, samarium, gold, and combinations thereof as disclosed in US 6,077,413.

Where the treatment agent comprises a radioisotope, any suitable method of applying the radioisotope, including those disclosed in the above-mentioned patents, may be used.

The invention may be used in a method of treating restenosis in a stented region of a bodily vessel where the stented region of the bodily vessel has a first stent therein with a lumen therethrough. The method comprises the steps of disposing a second stent in the lumen of the first stent and implanting the second stent in the lumen of the first stent. At least a portion of the second stent has a wall thickness of less than 0.001 inches. Even more desirably, the wall thickness is 0.0005 inches or less and most desirably, the wall thickness is 0.00025 inches or less.

Desirably, where the second stent has a tubular surface with openings therethrough, the ratio of the area of the surface to the area of the openings when the second stent is expanded is at least 3:7, more desirably, at least 1:1 and most desirably at least 3:2.

The invention may be used in a method of treating restenosis in a stented region of a bodily vessel, the stented region of the bodily vessel having a first stent therein with a lumen therethrough, the method comprising the steps of disposing and implanting a second stent in the lumen of the first stent. The second stent has a tubular surface with openings therethrough. The ratio of the area of the surface to the area of the openings when the second stent is expanded is at least 3:7, desirably, at least 1:1 and most desirably at least 3:2.

Any of the methods disclosed above may employ any of the second stents disclosed above, whether self-expanding, mechanically expandable or otherwise expandable.

Where the stent comprises a plurality of struts, such as that shown in Figs. 3 and 4, the struts are desirable of a thickness of 0.001 inches or less, more desirably 0.0005 inches or less and most desirably, 0.00025 inches or less. The thinner stents may be desirably be provided in a foil stent as discussed above.

The invention may be used in methods of implanting a second stent within an already implanted first stent which may be practiced after the first stent has been implanted for at least a month, two months, six months, a year or more. More generally, the second stent may be implanted at any time subsequent to the deployment of the first stent. Typically, the method will be carried out subsequent to restenosis of the stented region of the vessel.

Any of the methods discussed herein may also include one or more treatment steps in which the stented region of the vessel is treated to reduce or eliminate restenosis, desirably, prior to insertion of the second stent in the first stent. Although specific treatment processes are discussed briefly below, any suitable treatment process for reducing or eliminating restenosis may be used.

Balloon angioplasty, a technique in which a balloon is expanded in a stenotic region of a vessel, may be used. Additional details concerning balloon angioplasty may be found in US 6,010,480. Another technique which may be employed involves the use of a thermal catheter comprising a balloon as disclosed in US 4,799,479. Yet another technique which may be used employs both a balloon and a laser as disclosed in US 5,741,246. Other suitable techniques for reducing restenosis involve the use of a laser catheter as disclosed in US 6,106,51. The laser may be an excimer laser, Nd:YAG, holmium, CO₂ laser or any other suitable laser.

Techniques in which plaque is mechanically removed from the stented region may also be employed. US 5,941,869, for example, describes a debulking process which employs a catheter system having a stenotic material removal mechanism mounted on a distal portion of an elongated inner catheter. Rotational atherectomy is yet another technique that may be used. Rotational atherectomy incorporates a rotary file or burr into the distal portion of a catheter to remove plaque. Transluminal extraction catheter atherectomy involves the use of a catheter with tip-mounted cutting blades to remove plaque.

Another treatment modality involves the use of radiation treatment, as disclosed in US 5,833,593.

Yet another approach involves treatment with a drug which modulates cell growth into the target artery to inhibit the proliferation of smooth muscle cells. In particular, antiplatelet agents such as aspirin and dipyridamole, and anticoagulants such as heparin, have inhibited platelet aggregation and thrombus formation to a limited degree, thereby reducing the risk of early occlusion. Other suitable treatment agents are disclosed above.

The discussed combinations and methods may be used for coronary arteries, peripheral arteries, arteries of the neck and intracranial arteries. More generally, the combinations and methods may be used for any vessel of the human body including but not limited to arteries, veins, biliary ducts, urethras, fallopian tubes, bronchial tubes, the trachea and the esophagus.

The above disclosure is intended to be illustrative and not exhaustive.

## Claims

1. A second stent (112) adapted to be disposed within an implanted first stent (104), **characterized in that** at least a portion of the second stent (112) has a wall thickness of less than 0.0025 cm (0.001 inch).

2. The stent of claim 1 wherein the second stent (112) is self-expanding.

3. The stent of claim 2 wherein the second stent (112) is made of a shape memory material.

4. The stent of claim 1 wherein the second stent (112) is a foil stent.

5. The stent of claim 4 wherein the second stent (112) is only one layer of foil thick.

6. The stent of claim 1 wherein the second stent (112) comprises a bioabsorbable membrane.

7. The stent of claim 6 wherein the bioabsorbable membrane comprises a treatment agent.

8. The stent of claim 1 wherein the second stent (112) comprises a liner (128).

9. The stent of claim 8 wherein the liner (128) is expanded PTFE.

10. The stent of claim 1 wherein the second stent (112) comprises at least a first support (132) and a second support (132), and a liner (128) extending between the first support (132) and the second support (132).

11. The stent of claim 10 wherein the first and second supports (132) are hoops.

12. The stent of claim 10 wherein the liner (128) has a wall thickness of less than 0.003 cm (0.001 inches).

13. The stent of claim 1 wherein the second stent (112) is attachable to the first stent.

14. The stent of claim 13 wherein the second stent (112) is attachable to the first stent via a hook.

15. The stent of claim 13 wherein the second stent (112) is attachable to the first stent (104) via a plurality of hooks.

16. The stent of claim 13 wherein the second stent (112) may be bonded to the first stent (104).

17. The stent of claim 1 where the second stent (112) may apply an outward force against the first stent (104).

18. The stent of claim 1 wherein the second stent (112) is a spiral stent with a liner (128).

19. The stent of claim 1 wherein the second stent (112) has a tubular surface with openings therethrough, the second stent (112) when expanded having a ratio of the area of the surface to the area of the openings at least 3: 7.

20. The stent of claim 1 wherein the second stent (112) has a tubular surface with openings (111) therethrough, the second stent (112) when expanded having a ratio of the area of the surface to the area of the openings (111) at least 1:1.

21. The stent of claim 1 wherein the second stent (112) has a tubular surface with openings (111) therethrough, the second stent (112) when expanded having a ratio of the area of the surface to the area of the openings (111) at least 3:2.

## Patentansprüche

1. Ein zweiter Stent (112), der dafür geeignet ist, innerhalb eines implantierten ersten Stents (104) angeordnet zu werden, **dadurch gekennzeichnet, dass** zumindest ein Teil des zweiten Stents (112) eine Wandstärke von weniger als 0.0025 cm (0.001 inch) besitzt.

2. Der Stent gemäß Anspruch 1, wobei der zweite Stent (112) selbstexpandierend ist.

3. Der Stent gemäß Anspruch 2, wobei der zweite Stent (112) aus einem formspeichernden Material hergestellt ist.

4. Der Stent gemäß Anspruch 1, wobei der zweite Stent (112) ein Folienstent ist.

5. Der Stent gemäß Anspruch 4, wobei der zweite Stent (112) nur so dick wie eine Folienschicht ist.

6. Der Stent gemäß Anspruch 1, wobei der zweite Stent (112) eine biologisch absorbierbare Membran aufweist.

7. Der Stent gemäß Anspruch 6, wobei die biologisch absorbierbare Membran einen Wirkstoff für die Behandlung aufweist.

8. Der Stent gemäß Anspruch 1, wobei der zweite Stent (112) einen Einsatz (128) aufweist.

9. Der Stent gemäß Anspruch 8, wobei der Einsatz (128) aus expandiertem PTFE besteht.

10. Der Stent gemäß Anspruch 1, wobei der zweite Stent (112) zumindest eine erste Halterung (132) und eine zweite Halterung (132) und einen Einsatz (128) umfasst, der sich zwischen der ersten Halterung (132) und der zweiten Halterung (132) erstreckt.

11. Der Stent gemäß Anspruch 10, wobei die erste und zweite Halterung (132) Ringe sind.

12. Der Stent gemäß Anspruch 10, wobei der Einsatz (128) eine Wandstärke von weniger als 0.003 cm (0.001 inches) besitzt.

13. Der Stent gemäß Anspruch 1, wobei der zweite Stent (112) am ersten Stent befestigbar ist.

14. Der Stent gemäß Anspruch 13, wobei der zweite Stent (112) am ersten Stent mittels eines Hakens befestigbar ist.

15. Der Stent gemäß Anspruch 13, wobei der zweite Stent (112) am ersten Stent (104) mittels einer Vielzahl von Haken befestigbar ist.

16. Der Stent gemäß Anspruch 13, wobei der zweite Stent (112) mit dem ersten Stent (104) verklebt werden kann.

17. Der Stent gemäß Anspruch 1, wobei der zweite Stent (112) eine nach außen gerichtete Kraft gegen den ersten Stent (104) anwenden kann.

18. Der Stent gemäß Anspruch 1, wobei der zweite Stent (112) ein spiralförmiger Stent mit einem Einsatz (128) ist.

19. Der Stent gemäß Anspruch 1, wobei der zweite Stent (112) eine rohrförmige Oberfläche mit Öffnungen, die durch diese hindurchführen, besitzt, und der zweite Stent (112), wenn er expandiert ist, ein Verhältnis von zumindest 3 : 7 zwischen dem Oberflächenbereich und dem Bereich der Öffnungen aufweist.

20. Der Stent gemäß Anspruch 1, wobei der zweite Stent (112) eine rohrförmige Oberfläche mit Öffnungen (111), die durch diese hindurchführen, besitzt, und der zweite Stent (112), wenn er expandiert ist, ein Verhältnis von zumindest 1 : 1 zwischen dem Oberflächenbereich und dem Bereich der Öffnungen (111) aufweist.

21. Der Stent gemäß Anspruch 1, wobei der zweite Stent (112) eine rohrförmige Oberfläche mit Öffnungen (111), die durch diese hindurchführen, besitzt, und der zweite Stent (112), wenn er expandiert ist, ein Verhältnis von zumindest 3 : 2 zwischen dem Oberflächenbereich und dem Bereich der Öffnungen (111) aufweist.

## Revendications

1. Seconde prothèse endovasculaire (112) conçue pour être disposée dans une première prothèse endovasculaire (104) implantée, **caractérisée en ce qu'**au moins une partie de la seconde prothèse endovasculaire (112) présente une épaisseur de paroi inférieure à 0,0025 cm (0,001 pouce).

2. Prothèse endovasculaire selon la revendication 1, dans laquelle la seconde prothèse endovasculaire (112) est auto-expansive.

3. Prothèse endovasculaire selon la revendication 2, dans laquelle la seconde prothèse endovasculaire (112) est constituée d'un matériau à mémoire de forme.

4. Prothèse endovasculaire selon la revendication 1, dans laquelle la seconde prothèse endovasculaire (112) est une prothèse endovasculaire en feuille.

5. Prothèse endovasculaire selon la revendication 4, dans laquelle la seconde prothèse endovasculaire (112) a une épaisseur de seulement une couche de feuille.

6. Prothèse endovasculaire selon la revendication 1, dans laquelle la seconde prothèse endovasculaire (112) comprend une membrane bioabsorbable.

7. Prothèse endovasculaire selon la revendication 6, dans laquelle la membrane bioabsorbable comprend un agent de traitement.

8. Prothèse endovasculaire selon la revendication 1, dans laquelle la seconde prothèse endovasculaire (112) comprend un fond protecteur (128).

9. Prothèse endovasculaire selon la revendication 8, dans laquelle le fond protecteur (128) est du PTFE expansé.

10. Prothèse endovasculaire selon la revendication 1, dans laquelle la seconde prothèse endovasculaire (112) comprend au moins un premier support (132) et un second support (132) et un fond protecteur (128) s'étendant entre le premier support (132) et le second support (132).

11. Prothèse endovasculaire selon la revendication 10, dans laquelle le premier et le second supports (132) sont des anneaux.

12. Prothèse endovasculaire selon la revendication 10, dans laquelle le fond protecteur (128) a une épaisseur de paroi inférieure à 0,003 cm (0,001 pouce).

13. Prothèse endovasculaire selon la revendication 1, dans laquelle la seconde prothèse endovasculaire (112) peut être fixée à la première prothèse endovasculaire.

14. Prothèse endovasculaire selon la revendication 13, dans laquelle la seconde prothèse endovasculaire (112) peut être fixée à la première prothèse endovasculaire à l'aide d'un crochet.

15. Prothèse endovasculaire selon la revendication 13, dans laquelle la seconde prothèse endovasculaire (112) peut être fixée à la première prothèse endovasculaire (104) à l'aide d'une pluralité de crochets.

16. Prothèse endovasculaire selon la revendication 13, dans laquelle la seconde prothèse endovasculaire (112) peut être liée à la première prothèse endovasculaire (104).

17. Prothèse endovasculaire selon la revendication 1, dans laquelle la seconde prothèse endovasculaire (112) peut appliquer une force vers l'extérieur contre la première prothèse endovasculaire (104).

18. Prothèse endovasculaire selon la revendication 1, dans laquelle la seconde prothèse endovasculaire (112) est une prothèse endovasculaire en spirale comportant un fond protecteur (128).

19. Prothèse endovasculaire selon la revendication 1, dans laquelle la seconde prothèse endovasculaire (112) a une surface tubulaire traversée par des ouvertures, la seconde prothèse endovasculaire (112) ayant, lorsqu'elle est expansée, un rapport de la superficie de la surface à la superficie des ouvertures d'au moins 3/7.

20. Prothèse endovasculaire selon la revendication 1, dans laquelle la seconde prothèse endovasculaire (112) a une surface tubulaire traversée par des ouvertures (111), la seconde prothèse endovasculaire (112) ayant, lorsqu'elle est expansée, un rapport de la superficie de la surface à la superficie des ouvertures (111) d'au moins 1/1.

21. Prothèse endovasculaire selon la revendication 1, dans laquelle la seconde prothèse endovasculaire (112) a une surface tubulaire traversée par des ouvertures (111), la seconde prothèse endovasculaire (112) ayant, lorsqu'elle est expansée, un rapport de la superficie de la surface à la superficie des ouvertures (111) d'au moins 3/2.
